# EUROPEAN PATENT APPLICATION

(11) **EP 3 865 043 A1**
(43) Date of publication of application: **18.08.2021**
(21) Application number: 19886486.0
(22) Date of filing: 13.11.2019
(51) Int. Cl.: A61B 1/00, A61B 17/34

(54) **TROCAR**

(30) Priority: 19.11.2018 JP 2018216694
(71) Applicant: Nikkiso Co., Ltd., Tokyo 150-6022 (JP)
(72) Inventor: IGARASHI Tatsuo, Chiba-shi, Chiba 263-8522 (JP); SHIMOMURA Yoshihiro, Chiba-shi, Chiba 263-8522 (JP); MATSUNAGA Yoshihisa, Chiba-shi, Chiba 263-8522 (JP)
(74) Representative: Wunderlich & Heim Patentanwälte Partnerschaftsgesellschaft mbB
(86) International application number: PCT/JP2019/044595
(87) International publication number: WO 2020/105528

(57) **Abstract**

A trocar (10) is used by inserting a distal end thereof into a body cavity of a patient. The trocar (10) includes: an inner tube (20) that has a center duct line (16); an outer tube (22) that is disposed so as to cover an outer periphery of the inner tube (20) and has a front end that is positioned rearward of a front end of the inner tube (20); an annular flow path (18) that is formed between the inner tube (20) and the outer tube (22) and has an ejection port (30) at the front end position of the outer tube (22); and a flow control member (40) that is provided on the inner tube (20) at a position forward of the front end of the outer tube (22) and facing the ejection port (30), the flow control member (40) having an inclined surface located at the rear end that slants radially outward.

## Description

### TECHNICAL FIELD

The present disclosure relates to a trocar which is used by inserting a tip side thereof into a body cavity of a patient.

### BACKGROUND

Some surgeries are performed with equipment such as an endoscope (surgical equipment or surgical instrument) being inserted into a body cavity of a patient. In this case, a trocar is used as a guiding tube for the equipment to be inserted.

A trocar with a perfusion function of the related art described in Patent Literature 1 has a structure in which an access port, a water injection port, and a water absorption port of the equipment are integrated. For example, in surgery which is performed in a manner similar to an in-water endoscopic surgery, three functions are provided to the port, so as to realize suppression of a number of incisional wounds. In addition, a cross sectional area of a tip opening of the water absorption port is an area greater than or equal to five times a cross sectional area of an opening on an ejection side, so that a flow rate at the outflow side is fast, and contamination such as blood in a surgical field (an area of surgery) can thereby be effectively removed.

Patent Literature 2 discloses a body wall contact type water tank having functions of a fluid flow entrance to both the incisional wound and the body cavity, in a surgical form such as the in-water endoscopic surgery described above. In this example structure, because there is a storage space for fluid outside of the body cavity and a body cavity flow entrance of the fluid is relatively wide, diffusion of bleeding of the like, which occurs in the body cavity, in the fluid can be suppressed, and a new perfusion solution can be continuously supplied.

Patent Literature 3 discloses surgical equipment in which an injection direction when the perfusion solution is injected into the body cavity is changed to a direction at a right angle to a tube axis of the trocar, so as to avoid direct flow of a jet to the area of surgery. In other words, a collar element is provided opposing an ejection port, and the direction of the flow is changed by the collar element.

### CITATION LIST

### PATENT LITERATURE

Patent Literature 1: JP 2012-81191 A
Patent Literature 2: JP 2014-61132 A
Patent Literature 3: JP 2018-86168 A

### SUMMARY

### TECHNICAL PROBLEM

In Patent Literature 1, because the flow rate at an opening on the ejection side is relatively fast, when, for example, a body cavity which is a surgical target is wide and an amount of contamination included in the perfusion solution in the area of surgery is large, the contamination diffuses throughout a wide range in the area of surgery, and thus, a field of view in the area of surgery tends to be easily blocked.

In Patent Literature 2, because a water tank is provided, a relatively large incisional wound is necessary. In addition, with in-water endoscopic surgery, a surgical instrument for the endoscopic surgery may be used, but when the surgical instrument is inserted into a wide opening, it may be difficult to fix a fulcrum of the equipment.

In Patent Literature 3, when a large amount of perfusion solution is used, the flow rate becomes fast, and the flow direction cannot be sufficiently controlled. In particular, because a diameter of the collar element is set smaller than an inner size of an outer tube in order to maintain an abdominal wall penetration capability (an ability of penetrating an abdominal wall) of the trocar, there may be cases in which the flow direction control cannot be sufficiently performed.

An advantage of the present disclosure lies in improvement of controllability of the flow of fluid to be supplied with the trocar.

### SOLUTION TO PROBLEM

According to one aspect of the present disclosure, there is provided a trocar which is used by a tip side thereof being inserted into a body cavity of a patient, the trocar comprising: an inner tube that has a center duct line; an outer tube that is placed covering an outer circumference of the inner tube and in which a front end is positioned in the rear of a front end of the inner tube; a flow path that is formed between the inner tube and the outer tube, and that has an ejection port at a position of the front end of the outer tube; and a flow control member that is provided at a position in front of the front end of the outer tube and opposing the ejection port, wherein the flow control member has an inclined surface which is slanted rearward toward an outer side in a radial direction.

In another configuration, the flow control member may have a concave surface at a portion opposing the ejection port.

In another configuration, an outer size of the flow control member may be greater than an outer size of the ejection port.

In another configuration, the flow path may be an annular flow path, and the ejection port may be an annular ejection port.

In another configuration, the flow control member may have a cone shape which widens from an entirety of the outer circumference of the inner tube toward an outer side in the radial direction and in a slanted rearward direction, so that a concave surface is formed on an upper surface of the flow control member.

In another configuration, a spacer may be provided between an outer circumference of the inner tube and an inner circumference of the outer tube, which maintains a spacing between the outer circumference of the inner tube and the inner circumference of the outer tube.

In another configuration, a plurality of the spacers may be provided in an equal interval in an outer circumferential direction of the inner tube.

Definition of the terms used in the present specification and in the claims are as follows.

A "tube axis" is an axis which is a center line of the inner tube and the outer tube.

A "in front of' is a front side on the tube axis, and is a direction of a forward movement of the trocar when the trocar is inserted into the body cavity along the tube axis.

A "in the rear of' is a rear side on the tube axis, and is a direction of a backward movement of the trocar along the tube axis.

A "front end" is an end at the front side.

A "rear end" is an end at the rear side.

An "upper surface" is a surface which opposes the ejection port.

A "radial direction" is a direction of a line segment drawn from the tube axis of the inner tube and the outer tube, orthogonal to the circumferences thereof.

### ADVANTAGEOUS EFFECTS

According to the present disclosure, a flow of the fluid effused from the ejection port may be controlled and may be effectively moved away from the area of surgery.

### BRIEF DESCRIPTION OF DRAWINGS

FIG. 1 is a diagram showing an outer appearance of a structure of a trocar according to an embodiment of the present disclosure.
FIG. 2 is a diagram showing a cross section of the trocar of FIG. 1, in the tube axis direction.
FIG. 3 is a diagram showing a structure of an inner tube, where (a) is a front view, and (b) is a cross sectional view along a line B-B.
FIG. 4 is a diagram showing a structure in which an outer tube 22 is placed at an outer side of the inner tube, where (a) is a front view (with the outer tube shown with two-dots-and-chain line), and (b) is a cross sectional view along a line B-B.
FIG. 5 is an explanatory diagram of a flow of fluid from an ejection port.
FIG. 6A is a photograph showing a state in which water is filled in a beaker, a trocar is inserted in the water, and a fluid including an ink is effused.
FIG. 6B is a diagram schematically showing the photograph of FIG. 6A.
FIG. 7 is a diagram showing an example configuration in which a female screw portion is provided on an inner wall of a gripping portion, and a male screw portion is provided at a corresponding position at a periphery of the inner tube.
FIG. 8 is a diagram showing an example configuration in which a projection is provided on an inner wall of the gripping portion, and a concave is provided at a corresponding position at a periphery of the inner tube.
FIG. 9 is a diagram showing another example structure (structure with a flat surface portion) of a flow control member.
FIG. 10 is a diagram showing another example structure (petal shape) of the flow control member.
FIG. 11 is a diagram showing another example structure (umbrella shape) of the flow control member.

### DESCRIPTION OF EMBODIMENTS

An embodiment of the present disclosure will now be described with reference to the drawings. The present disclosure is not limited to the embodiment described herein.

### [Overall Structure]

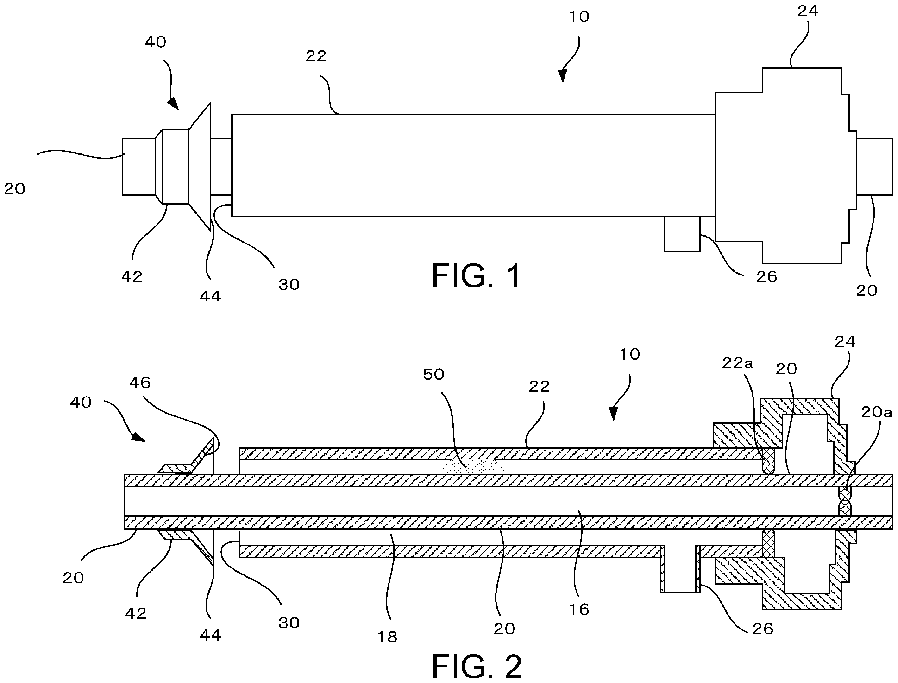

FIG. 1 shows an outer appearance of a structure of a trocar 10 according to the present embodiment, and FIG. 2 is a cross sectional diagram in a tube axis direction.

The trocar 10 has a double tube structure in which two circular cylindrical tubes are placed concentrically, and has a center duct line 16, and an annular flow path 18. The center duct line 16 is a hollow duct line inside an inner tube 20, penetrates through the entirety of the inner tube 20, and has respective ends opened. Thus, the center duct line 16 is used as a passage for inserting and removing a surgical device such as an endoscope. On the center duct line 16, a valve 20a is placed, so as to prevent flowing out of fluid from a rear end of the center duct line 16 to the outside, while enabling insertion and removal of the surgical device.

A length of an outer tube 22 is shorter than the inner tube 20, and both a front end and a rear end thereof are positioned at a side that is further inward than respective ends of the inner tube 20. The annular flow path 18 is formed with a vacant space between an inner circumferential surface of the outer tube 22 and an outer circumferential surface of the inner tube 20. The annular flow path 18 has the front end opened, but the rear end of the outer tube 22 extends to an outer circumference of the inner tube 20 to close the annular flow path 18. In this example configuration, the inner tube 20 can move with respect to the outer tube 22 in the tube axis direction, and a valve 22a is placed at the rear end of the outer tube 22 to close the rear end of the annular flow path 18. The annular flow path 18 does not need to have a perfect annular shape.

A gripping portion 24 is provided, covering an outer side of a rear portion of the outer tube 22. A front end of the gripping portion 24 is fixed on an outer circumference of the outer tube 22, and a hole passing through the inner tube 20 is provided at a rear end of the gripping portion 24. That is, the inner tube 20 penetrates through the rear end of the gripping portion 24, and can move with respect to the gripping portion 24 ion the tube axis direction.

At a position on the outer tube 22 near the gripping portion 24, a fluid inflow tube 26 which is in communication with the annular flow path 18 is placed toward an outer side in a radial direction. A fluid is supplied from the fluid inflow tube 26 toward the annular flow path 18.

The front end of the annular flow path 18 ends at a rear side of the front end of the inner tube 20, to form an ejection port 30 which is opened and which has an annular shape. In other words, the annular ejection port 30 is formed at a front end position of the outer tube 22. The fluid (perfusion solution) to be supplied to the annular flow path 18 is effused from the ejection port 30.

At a position at a front side of the ejection port 30 and opposing the ejection port 30, a flow control member 40 is placed. The flow control member 40 has a base 42 having a circular cylindrical shape, and a saucer portion 44 having a cone shape (an inverted circular cone trapezium shape) which widens from an upper end (rear side) of the base 42 toward an outer side in the radial direction and toward a slanted rearward direction. An upper surface (surface opposing the ejection port 30) of the saucer portion 44 is a concave surface 46. The base 42 is attached on the outer circumference of the inner tube 20, and at a position which is a predetermined distance in front from the ejection port 30. Because of this structure, the fluid effused from the ejection port 30 hits the concave surfaced 46 of the flow control member 40, and the direction of the flow thereof is changed.

The perfusion solution is suctioned from a suctioning cannula placed at a periphery of a surgical site so that a pressure at the periphery is maintained at a desired pressure.

The inner tube 20, the outer tube 22, the gripping portion 24, and the like are desirably formed from a plastic such as polycarbonate, but may alternatively be formed from a metal such as the stainless steel. The flow control member 40 desirably has a certain degree of flexibility, and is desirably formed from a silicon resin, or other soft plastic materials. The valves 20a and 22a are also desirably formed from the plastic.

### [Structure of Spacer]

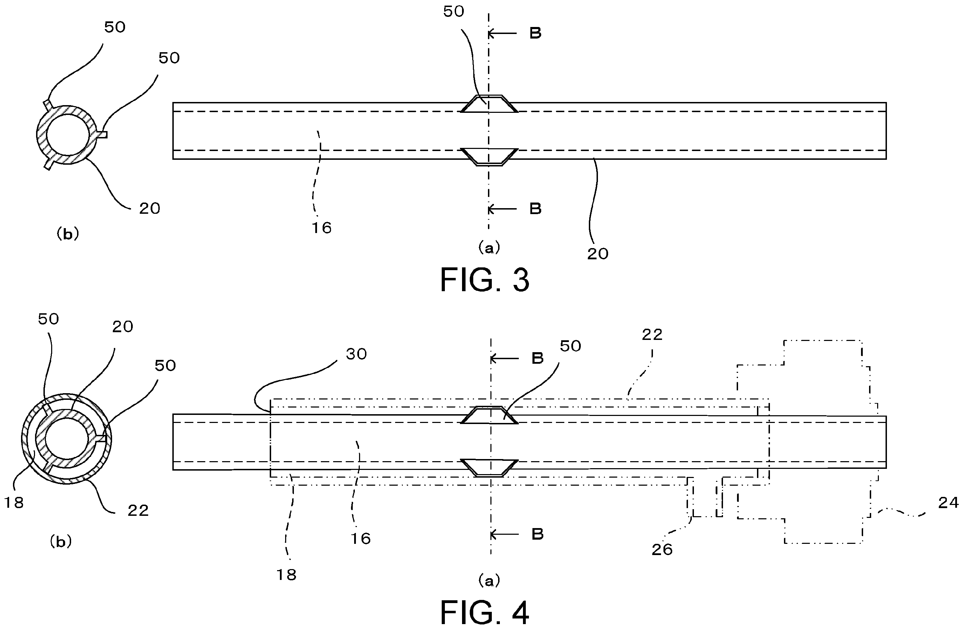

FIG. 3 shows a structure of the inner tube 20, where (a) is a front view and (b) is a cross sectional view along a line B-B. FIG. 4 shows a structure in which the outer tube 22 is placed at an outer side of the inner tube 20, where (a) is a front view (showing the outer tube 22 with a two-dots-and-chain line), and (b) is a cross sectional view along a line B-B.

As shown, at an outer circumference of a middle portion of the inner tube 20 in the tube axis direction, a spacer 50 of a plate shape is provided, which has a predetermined length in the tube axis direction and which extends toward an outer side in the radial direction. In the illustrated example configuration, three spacers 50 are placed on the outer circumferential surface of the inner tube 20 at equal intervals. A cross section of the spacer 50 passing through the tube axis has a trapezoid shape, an inner end of the spacer 50 is fixed on the outer circumferential surface of the inner tube 20, and an outer end of the spacer 50 is in contact with the inner circumferential surface of the outer tube 22. By placing such a spacer 50, the annular flow path 18 in the space between the inner tube 20 and the outer tube 22 can be maintained, and an orientation of the trocar 10 can be stabilized.

When a position of placement of the spacer 50 is near the rear end of the outer tube 22, the effect of maintaining the space is small, and when the position of placement is near the ejection port 30, the spacer 50 may affect the flow of the fluid effused from the ejection port 30. Therefore, the spacer 50 is desirably placed near an approximate center of the inner tube 20 in the length direction. The number of the spacers 50 may be two or more, but when the number is too large, the cross section of the annular flow path 18 becomes small. Therefore, an appropriate number of the spacers 50 is about 3.

For the spacer 50, a material which elastically deforms may be desirably employed, such as a plastic. Further, a concave groove for housing the outer end of the spacer 50 may be provided on the inner circumferential surface of the outer tube 22.

### [Flow Control]

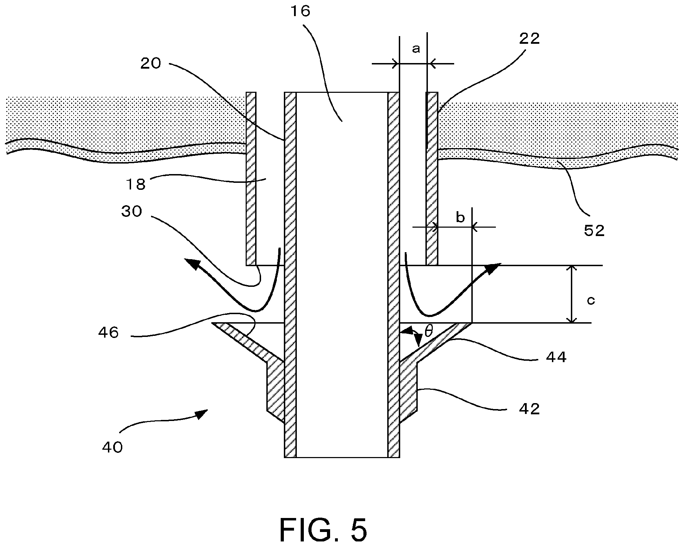

FIG. 5 is an explanatory diagram of a flow of fluid from the ejection port 30. The trocar 10 is inserted in a manner to penetrate through the body wall, and a front end side thereof reaches an inside of the body cavity. For example, a peritoneum 52 is an inner wall of a body cavity, and fluid (perfusion solution) is filled in the body cavity.

The fluid from the annular flow path 18 is effused from the entirety of the annular ejection port 30 toward the front side in the tube axis direction. On the upper surface (surface opposing the ejection port 30) of the saucer portion 44 of the flow control member 40, the concave surface 46 is formed. Therefore, the fluid effused from the ejection port 30 moves toward the front side in the tube axis direction, as shown by an arrow in FIG. 5, hits the concave surface 46 of the saucer portion 44 of the flow control member 40, where the direction of the flow thereof is changed, and flows in a radiating direction toward a slanted upward direction. In particular, the outer end of the saucer portion 44 is set at an side that is further out in the radial direction than the inner circumference of the outer tube 22. Therefore, it is possible to prevent the flow of the fluid, effused in the tube axis direction from the ejection port 30 of the annular flow path 18, from directly flowing in the tube axis direction. Because the flow of the fluid effused from the ejection port 30 contacts the fluid at the periphery, the flow is disturbed. Thus, when the outer end of the saucer portion 44 is set to be equal to or smaller than the inner size of the outer tube 22, the flow in the tube axis direction cannot be effectively prevented. In the present embodiment, the outer size of the saucer portion 44 is set greater than the inner size of the outer tube 22, so that the flow in the tube axis direction can be effectively prevented, and even when a fluid is to flow with a high flow rate, the fluid flow toward the area of surgery can be blocked and can be moved away.

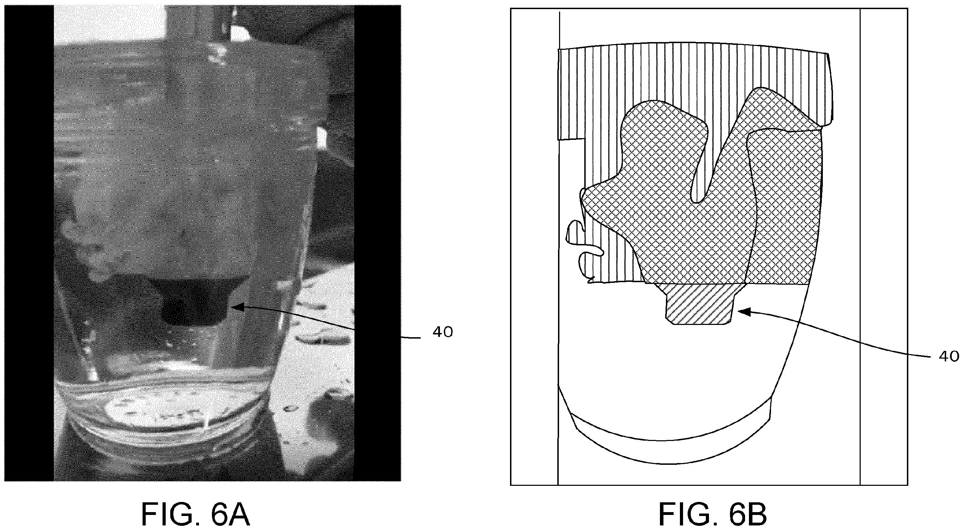

FIG. 6A is a photograph of a state in which water is filled in a beaker, the trocar 10 is inserted into the water, and a fluid including an ink is effused. FIG. 6B is a diagram schematically showing a spreading state of the fluid including the ink. As shown, it can be understood that by changing the flow of the fluid moving in the tube axis direction to a slanted upward direction with the flow control member 40, the disturbance of the fluid flow can be prevented at the area of surgery at a lower side of the flow control member 40.

In the present embodiment, the outer size of the flow control member 40 is set greater than the outer size of the outer tube 22, but the outer size of the flow control member 40 may alternatively be smaller than the outer size of the outer tube 22 so long as the outer size of the flow control member 40 is greater than the inner size of the outer tube 22 (that is, the outer size of the ejection port 30). That is, b in FIG. 5 may be set to between 0 and a few mm.

### [Other Structures]

### <Adjustment of Distance between Ejection Port 30 and Flow Control Member 40>

A relative position in the tube axis direction of the outer tube 22 with respect to the inner tube 20 may be set adjustable so that the position of the flow control member 40 in the tube axis direction is adjustable, to enable adjustment of a distance c in FIG. 5, between the ejection port 30 and the flow control member 40. For example, the distance c may be set to a few mm. In this manner, the distance may be determined based on the use.

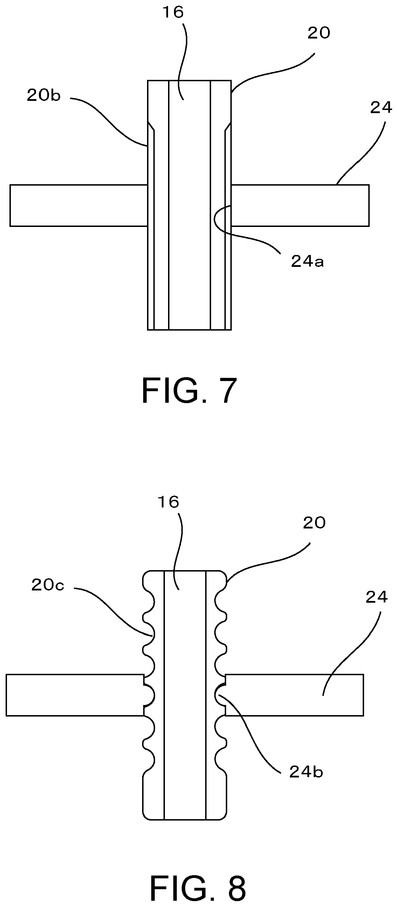

FIG. 7 shows an example configuration in which a female screw portion 24a is provided on an inner wall of a gripping portion 24, and a male screw portion 20b is provided at a corresponding position on an outer circumference of the inner tube 20. With such a structure, the inner tube 20 may be rotated with respect to the gripping portion 24, to move the inner tube 20 in a front and rear direction in the tube axis direction, and a distance between the flow control member 40 fixed on the inner tube 20 and the ejection port 30 can thereby be adjusted.

FIG. 8 shows another example configuration for the gripping portion 24 and the outer circumference of the inner tube 20. In this example configuration, a protrusion 24b having a semicircular cross section and protruding toward an inner side in the radial direction is provided on the inner wall of the gripping portion 24, and a recess 20c of a corresponding shape is provided on the outer circumference of the inner tube 20. With such a configuration, the inner tube 20 may be moved in the front-and-rear direction in the tube axis direction so that the position of the inner tube 20 with respect to the outer tube 22 can be changed, and a distance between the ejection port 30 and the flow control member 40 fixed on the inner tube 20 can thereby be adjusted.

### <Flow Control Portion>

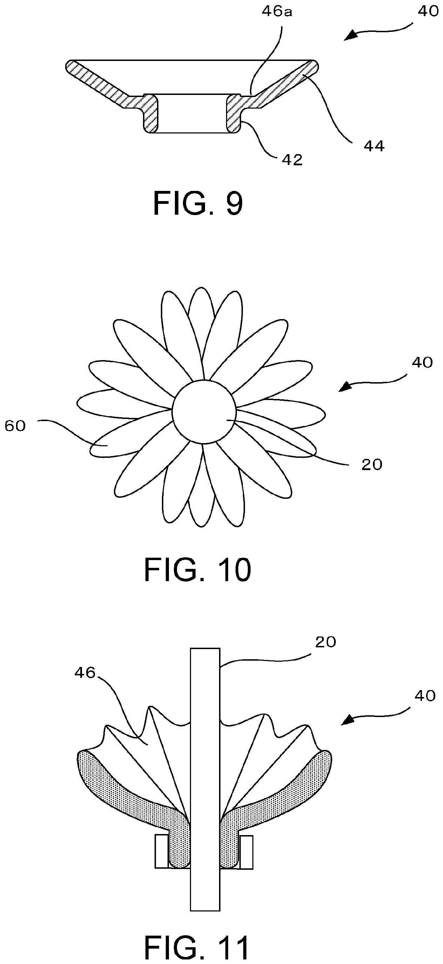

FIG. 9 shows another example configuration of the flow control member 40. In this example configuration, a flat surface portion 46a which is orthogonal to the tube axis is provided at an inner portion (side closer to the inner tube 20) of the concave surface 46 of the saucer portion 44 formed on the upper surface (surface opposing the ejection port 30) of the flow control member 40, and an inclined surface in a slanted upward direction is provided at an outer circumferential side of the flat surface portion 46a. With such a structure, the flow in the tube axis direction of the fluid effused from the ejection port 30 can be received, and the direction of this flow may be changed to a lateral direction (radial direction).

FIG. 10 shows yet another example configuration of the flow control member 40. In this example configuration, the flow control member 40 is formed from a plurality of (multiple) petal shaped members 60. Specifically, an upper surface of the petal shaped member 60 from a center part in a slanted upward direction forms the saucer portion 44. With such a configuration, the petal shaped member 60 may be opened and closed. Thus, the petal shaped member 60 may be closed when the trocar 10 is inserted into or removed from the body cavity. For the opening and closing of the petal shaped member 60, a suitable structure may be employed. For example, the petal shaped member 60 may be opened and closed by fixing the flow control member 40 on the outer tube 22 at a position a predetermined distance apart from the ejection port 30, and moving the inner tube 20 in the front and in the rear in the tube axis direction. Alternatively, the movement of the inner tube 20 in the tube axis direction may be realized by driving with a motor or the like.

FIG. 11 shows another example configuration of the flow control member 40. In this example configuration, the flow control member 40 has an umbrella shape. Similar to the above, with this configuration also, the flow control member 40 may be opened and closed.

### [Advantage of Embodiment]

According to the trocar 10 of the present embodiment, the outer size of the flow control member 40 is approximately equal to the outer size of the outer tube 22. Therefore, the trocar 10 can be easily inserted into the body cavity without changing the manipulations of the related art. Because the fluid flowing into the abdominal cavity does not directly reach the area of surgery, a continuous perfusion may be performed while maintaining the field of view.

That is, with the trocar 10 of the present embodiment, a flow close to a stable laminar flow may be created near the area of surgery, diffusion of bleeding during the surgery under the fluid (perfusion solution) can be prevented, and the field of view can continue to be secured.

### [Specific Specification]

Specific specifications will now be described.

The outer size of the trocar 10 is desirably set to a diameter of 12 ∼ 15 mm, which is equivalent to the outer size of a typical trocar. With such a configuration, an invasiveness similar to the trocar of the related art can be maintained.

As a result of a simulation by a computer and an experiment with a prototype, it was found that the space between the outer tube 22 and the inner tube 20 (width of the annular flow path 18, "a" in FIG. 5) is desirably about 1 mm. The outer size of the inner tube 20 of the trocar 10 used in the experiment was 9.5 mm, the inner size of the outer tube 22 was 12 mm, and the width of the annular flow path 18 was 1.25 mm.

A velocity of the fluid flow of the fluid effused from the ejection port 30 (perfusion solution) was set to approximately 300 ml/min. ∼ 1000 ml/min., the concave surface 46 was employed for the upper surface of the saucer portion 44 of the flow control member 40 in order to change the direction of the fluid flow, and the outer size was set to equivalent to or greater than the outer size of the outer tube 22.

Further, an angle θ of the inclined surface in the saucer portion 44 with respect to the tube axis direction was about 60 degrees, and was desirably in a range of 75 degrees ∼ 45 degrees.

Using a cray model of an inside of a pelvis and cow blood, a perfusion experiment simulating bleeding from a rear surface of a pubic bone was performed. The cow blood supplied from the trocar 10 flowed toward a suctioning cannula which was separately provided, and there was no blocking of the field of view due to the diffusion of the blood.

### REFERENCE SIGNS LIST

10 TROCAR; 16 CENTER DUCT LINE; 18 ANNULAR FLOW PATH; 20 INNER TUBE; 22 OUTER TUBE; 26 FLUID INFLOW TUBE; 30 EJECTION PORT; 40 FLOW CONTROL MEMBER; 42 BASE; 44 SAUCER PORTION; 46 CONCAVE SURFACE; 50 SPACER; 60 PETAL SHAPED MEMBER.

## Claims

1. A trocar which is used by a tip side thereof being inserted into a body cavity of a patient, the trocar comprising:
an inner tube that has a center duct line;
an outer tube that is placed covering an outer circumference of the inner tube and in which a front end is positioned in the rear of a front end of the inner tube;
a flow path that is formed between the inner tube and the outer tube, and that has an ejection port at a position of the front end of the outer tube; and
a flow control member that is provided at a position in front of the front end of the outer tube and opposing the ejection port, wherein
the flow control member has an inclined surface which is slanted rearward toward an outer side in a radial direction.

2. The trocar according to claim 1, wherein
the flow control member has a concave surface at a portion opposing the ejection port.

3. The trocar according to claim 2, wherein
an outer size of the flow control member is greater than an outer size of the ejection port.

4. The trocar according to any one of claims 1 to 3, wherein
the flow path is an annular flow path, and the ejection port is an annular ejection port.

5. The trocar according to any one of claims 1 to 4, wherein
the flow control member has a cone shape which widens from an entirety of an outer circumference of the inner tube toward an outer side in the radial direction and in a slanted rearward direction, so that a concave surface is formed on an upper surface of the flow control member.

6. The trocar according to any one of claims 1 to 5, wherein
a spacer is provided between an outer circumference of the inner tube and an inner circumference of the outer tube, which maintains a spacing between the outer circumference of the inner tube and the inner circumference of the outer tube.

7. The trocar according to claim 6, wherein
a plurality of the spacers are provided at equal intervals in an outer circumferential direction of the inner tube.
